# EUROPEAN PATENT APPLICATION

(11) **EP 4 056 224 A1**
(43) Date of publication of application: **14.09.2022**
(21) Application number: 21161990.3
(22) Date of filing: 11.03.2021
(51) Int. Cl.: A61N 1/05, A61N 1/36, A61N 1/375

(54) **ACTIVE IMPLANTABLE MEDICAL DEVICE FOR SENSING AN ELECTRIC SIGNAL OF AN ORGAN OF A HUMAN OR ANIMAL PATIENT**

(71) Applicant: BIOTRONIK SE & Co. KG, 12359 Berlin (DE)
(72) Inventor: RUMP, Jens, 12049 Berlin (DE); VAN OOYEN, André, 10717 Berlin (DE)
(74) Representative: Biotronik Corporate Services SE

(57) **Abstract**

The invention relates to an active implantable medical device for sensing an electric signal of an organ of a human or animal patient, comprising a processor, a memory unit (21), and a detection unit configured to detect an electric signal of an organ of a human or animal patient, wherein the detection unit comprises an elongated electrode body (3) extending between a proximal end and a distal end (13) of the active implantable medical device (1) and having at least one electrode pole (4), wherein the active implantable medical device (1) is configured to be implanted within a stomach (7) of the patient, the stomach (7) having a cardia (75), a fundus (74), a greater curvature (70), a lesser curvature (71), a pyloric antrum (73), and a stomach wall (72) with gastric mucosa (77) facing an interior of the stomach, with the elongated electrode body (3) being placed along the greater curvature (70). According to an aspect of the invention, the active implantable medical device (1) comprises a passive fixation element (5, 6) at at least one of the proximal end and the distal end (13) of the active implantable medical device (1), wherein the passive fixation element (5, 6) serves for a purely passive fixation of the active implantable medical device (1) at the gastric mucosa (77).

## Description

The present invention relates to an active implantable medical device for sensing an electric signal of an organ of a human or animal patient according to the preamble of claim 1, and to different medical uses of such a device according to the preambles of claims 13 and 14.

Implants for continuous monitoring of cardiac activity in the form of monitor implants are usually implanted subcutaneously in the shoulder or chest area of the patient at a comparable position to pacemakers or implantable defibrillators. Although the procedure is minimally invasive, the necessary skin incision and the formation of a pocket in the fatty or connective tissue that receives the implant are associated with a risk of infection. In addition, the finite life of an implant battery makes it necessary to replace the implant at regular intervals and thus to perform another operation.

Gastric pacemakers are similar in function to cardiac pacemakers and are usually implanted subcutaneously in the upper abdomen. The therapy is performed via electrical leads, which are usually attached to the stomach wall from the outside at the level of the antrum. Due to the high risk for the patient, strict indications are provided for this procedure.

An alternative position of an implant for monitoring the heart activity or a gastric pacemaker offers the inner stomach wall along the greater curvature. However, implants implanted there are actively anchored in the stomach wall by means of screws or other fixation elements. This can lead to considerable irritation of the stomach wall or the gastric mucosa.

US 2002/0165589 A1 discloses a device for diagnosing and treating gastric disorders. The device is tightly anchored within the gastric wall or even penetrates the gastric wall for anchoring purposes.

US 2004/0088033 A1 describes different embodiments of medical leads for use with implantable medical devices.

US 2008/0312712 A1 describes an implantable stimulation system for stimulating the heart, the phrenic nerve, the gastric system, or other tissue structures accessible via a patient's upper gastrointestinal system or airway. This stimulation system is thoroughly anchored by one or more anchor devices within the body structure into which it is implanted.

US 2010/0286745 A1 describes a fixation device for holding stimulation electrodes in electrical contact with the gastric wall. For this purpose, the fixation device penetrates the gastric wall.

US 2013/0345771 A1 describes a responsive gastrointestinal stimulation device that uses data sensed with a sensor for applying stimulation, adjusting stimulation or stopping stimulation of the gastrointestinal tract. The elements for fixating the stimulation device within the stomach penetrate the gastric wall.

US 8,135,470 B1 describes an implantable device being able to sense gastric data. For this purpose, the device comprises electrodes that may be surgically sutured onto the outer wall of the stomach or fixed by penetration of anchoring devices within the gastric tissue. Alternatively, surgical adhesives may be used to attach the electrodes.

WO 2018/104472 A1 describes a two-part implantable system, wherein one part of the system is fixed to the stomach wall of a patient by means of an anchor placed into the stomach wall.

It is an object of the present invention to provide a device which is easy to implant and whose implantation has fewer side effects than the devices known from prior art.

This object is achieved with an active implantable medical device for sensing an electric signal of an organ of a human or animal patient. Such a device comprises a processor, a memory unit, and a detection unit. The detection unit serves for detecting an electric signal of an organ of a human or animal patient. The detection unit comprises an elongated electrode body extending between a proximal end and a distal end of the active implantable medical device. The elongated electrode body has at least one electrode pole. The active implantable medical device is designed and arranged to be implanted within the stomach of the patient. The stomach comprises a plurality of physiologic substructures, namely, a cardia, a fundus, a greater curvature, a lesser curvature, a pyloric antrum, and a stomach wall. The stomach wall comprises different layers, wherein the gastric mucosa faces an interior of the stomach and is thus the innermost layer of the stomach wall. Upon implantation of the active implantable medical device, the elongated electrode body is placed along the greater curvature on or within the gastric mucosa.

Within the frame of this application an implant is to be understood as a device configured to be fixed or set securely or deeply within a human or animal body.

According to an aspect of the presently claimed invention, the active implantable medical device comprises a passive fixation element being positioned at the proximal end and/or at the distal end of the active implantable medical device. The passive fixation element serves for a purely passive fixation of the active implantable medical device at the gastric mucosa. Thus, in contrast to active fixation elements like screws or other anchoring elements, the passive fixation element does not project into the gastric wall nor penetrate the gastric wall, but rather lies on the innermost layer of the gastric wall and presses against the gastric wall. There is no chemical adhesive between the passive fixation element and the gastric wall to achieve the fixation of the active implantable medical device. Rather, the passive fixation of the active implantable medical device is achieved only by mechanical forces mainly applied by the passive fixation element.

Since the gastric mucosa has a highly wrinkled appearance leading to so-called gastric rugae, a purely passive fixation of the passive fixation element on the gastric wall can be easily achieved. The purely passive fixation is particularly tissue conserving and gentle to the gastric mucosa and the whole gastric wall. Irritations of the gastric mucosa and/or the gastric wall can be significantly reduced with respect to an active fixation of an implant within the gastric wall.

Implanting the active implantable medical device into the stomach of the patient is much less invasive than implanting a comparable medical device subcutaneously into other body parts. Due to the access via the esophagus, the inside of the stomach can be reached particularly easy and riskless. Placing the active implantable medical device under visual control by means of a gastroscope is possible. In case that a battery of the active implantable medical device needs to be exchanged, the whole device can be easily removed from its implantation site and exchanged by a novel device or re-implanted after having exchanged the battery.

Furthermore, in case of the stomach as implantation site, less restrictions with respect to size, shape, and mass of the implantable medical device exist as compared to implantable medical devices intended to be subcutaneously implanted, e.g., in the area of the shoulder.

The term "active implantable medical device" is well known to a person skilled in the art. An "active medical device" is typically defined to be any medical device relying for its functioning on a source of electrical energy or any source of power other than that directly generated by the human body or gravity.

An "active implantable medical device" is typically defined to be any active medical device which is intended to be totally or partially introduced, surgically or medically, into the human body or by medical intervention into a natural orifice, and which is intended to remain after the procedure.

Further details on active implantable medical devices can be found, e.g., in the consolidated text of the Council Directive 90/385/EEC of 20 June 1990 on the approximation of the laws of the Member States relating to active implantable medical devices with subsequent amendments in the version published on 11 October 2007. The consolidated text of this Council Directive is freely accessible under the following link: https://eurlex.europa.eu/legal-content/EN/TXT/?uri=CELEX:01990L0385-20071011

In an embodiment, the active implantable medical device is an implantable pulse generator (IPG), an implantable cardioverter-defibrillator (ICD), a device for cardiac resynchronization therapy (CRT), or an implantable cardiac monitor. An appropriate cardiac monitor is a loop recorder.

In an embodiment, the active implantable medical device is an implantable device for stimulating the diaphragm, the phrenic nerve, the gastric system, in particular the stomach, and/or other tissue structures accessible via a patient's upper gastrointestinal tract, in particular via the patient's stomach.

In an embodiment, the passive fixation element comprises or is entirely made of a flexible plastic, such as polyurethane or silicone. In an embodiment, the passive fixation element is realized in form of elastic wings or hooks. In doing so, care is taken that the specific shape of the passive fixation element ensures that the gastric mucosa is not penetrated by the passive fixation element. Rather, the passive fixation element is intended to only mechanically interact with the surface or the rugae of the gastric mucosa, but not to penetrate the gastric mucosa.

In an embodiment, the passive fixation element is positioned at the proximal end of the active implantable medical device. In its implanted state, the proximal end of the active implantable medical device is located in a gastric region near to the pyloric antrum (i.e., in a caudal region of the stomach). Likewise, the distal end of the active implantable medical device is intended to be located in or at the fundus, in particular adjacent to the cardia. Then, the elongated electrode body extends from the proximal end to the distal end of the active implantable medical device along the greater curvature. Such a position of the active implantable medical device is particularly appropriate for sensing and/or detecting cardiac signals and/or gastric signals of the patient.

In the implanted state of the active implantable medical device, the at least one electrode pole is in contact with the gastric mucosa in the region of the greater curvature or of a cranial section of the gastric wall (i.e., in the fundus). If the at least one electrode pole has achieved such a position within the stomach, both cardiac signals and gastric signals can be sensed in a particularly appropriate way.

In an embodiment, the active implantable medical device comprises two passive fixation elements. A first passive fixation element is disposed at the proximal end of the active implantable medical device, wherein a second passive fixation element is disposed at the distal end of the active implantable medical device. By such an arrangement, a particularly tight passive fixation of the active implantable medical device at the gastric mucosa is possible, thus avoiding undesired dislocations of the active implantable medical device.

In an embodiment, the active implantable medical device comprises a housing. Such a housing serves for accommodating the processor and the memory unit (and optionally other appliances such as a battery and/or an analogue-to-digital converter). The housing is operatively coupled to the elongated electrode body so that an electrode located within the elongated electrode body is able to transmit signals to components received within the housing or can be supplied with electric pulses provided by a component of the electric housing. In an embodiment, the housing is positioned between the proximal end of the elongated electrode body and the proximal end of the active implantable medical device. However, generally other positions of the housing are also conceivable. E.g., in another embodiment, the housing is positioned between a distal end of the elongated electrode body and the distal end of the active implantable medical device.

In an embodiment, the housing is positioned between the elongated electrode body and the passive fixation element.

In an embodiment, the housing has a cylindrical or a cuboid shape. It is typically hermetically sealed against its environment so as to protect the components received within the housing from any liquids such as body fluids being present outside the housing. Appropriate materials for manufacturing the housing are biocompatible materials such as titanium, polyether ether ketone (PEEK), polyurethane, platinum, silicone, tantalum, and medical grade stainless steel (such as MP35N).

In an embodiment, the elongated electrode body comprises at least two electrode poles, in particular 2 to 10, in particular 3 to 9, in particular 4 to 8, in particular 5 to 7 electrode poles, being spaced apart from each other by at least 10 mm, in particular by at least 15 mm, in particular by at least 20 mm, in particular by at least 25 mm, in particular by a distance lying in a range of from 10 mm to 25 mm, in particular of from 15 mm to 20 mm. The electrode poles can be realized as ring-shaped metal surfaces that are in direct contact to the gastric wall in the implanted state of the active implantable medical device. Generally, a plurality of electrode poles can be provided. They can be distributed over the whole length of the elongated electrode body. In an embodiment, the electrode poles are only located in a distal region of the active implantable medical device so as to contact the gastric mucosa in a cranial region of the gastric wall.

In an embodiment, the elongated electrode body comprises at least one, in particular 2 or 3 rope-shaped conductors that connect the processor or a control unit with the at least one electrode pole. In an embodiment, the number of conductors being present within the elongated electrode body corresponds to the number of electrode poles distributed along the electrode body.

In an embodiment, the elongated electrode body has a length lying in a range of from 15 cm to 50 cm, in particular of from 20 cm to 45 cm, in particular of from 25 cm to 40 cm, in particular of from 30 cm to 35 cm.

In an embodiment, the elongated electrode body has an elliptic cross-section. Then, it has a preferential direction with respect to the gastric wall so that an implantation of the active implantable medical device along the gastric wall in the region of the greater curvature is further facilitated.

In an embodiment, the at least one electrode pole is designed in form of a convex metal surface that is only present on that side of the elongated electrode body that is intended to face the gastric wall in the implanted state of the active implantable medical device.

In an embodiment, the elongated electrode body comprises at least three electrode poles. By such an arrangement, it is possible to capture cardiac signals by applying different cardiac recordings. Furthermore, it is possible to use at least two electrode poles being spaced apart from each other at a first distance to detect near field signals and to use at least two electrode poles being spaced apart from each other at a second, larger distance to detect far field signals. While near field signals typically are evoked by gastric activity (i.e., in the direct environment of the active implantable medical device), far field signals are typically evoked by the heart being located somewhat further away. By such a separate detection of near field signals and far field signals, it is particularly easy to subtract gastric signals from detected cardiac signals (that always contain to a certain amount gastric signals when sensed with an active implantable medical device located within the stomach of the patient and thus can also be denoted as signal mixture) so as to filter pure cardiac signals from the sensed signal mixture.

The use of at least three electrode poles additionally enables a mapping of cardiac signals according to their point of origin.

In an embodiment, the active implantable medical device comprises at least one or a plurality (two or more) of supportive passive fixation elements in addition to the passive fixation element. The supportive passive fixation element(s) is/are disposed along the elongated electrode body. They serve for an additional purely passive fixation of the active implantable medical device at the gastric mucosa so as to support the passive fixation achieved by the passive fixation element(s) located at the distal end and/or the proximal end of the active implantable medical device. These supportive passive fixation elements can be designed, e.g., as wings, hooks or in the shape of a disc. In an embodiment, they are produced from an elastic soft plastic such as polyurethane or silicone. Upon implantation of the active implantable medical device, they can claw themselves within the gastric rugae of the gastric wall so as to achieve a further mechanical-passive fixation of the active implantable medical device.

In an embodiment, the supportive passive fixation elements are folded towards an axis extending along the direction of extension of the elongated electrode body so that the maximum diameter of the elongated electrode body is not enlarged due to the supportive passive fixation elements. In an embodiment, this is achieved by at least one tapering provided within the elongated electrode body that is able to receive the folded supportive passive fixation elements. Upon implantation of the active implantable medical device, the supportive fixation elements are released from their folded position to a deployed position in which they project from the elongated electrode body.

The supportive passive fixation elements effect an even distribution of a contact pressure of the active implantable medical device onto the gastric wall due to the increased surface of the medical device and thus reduce the risk of an irritation of the gastric mucosa.

In an embodiment, the elongated electrode body comprises at least one Nitinol element. Such a Nitinol element provides the elongated electrode body with pseudo-elastic properties. In doing so, the Nitinol element serves for providing or enhancing a contact pressure of the elongated electrode body against the gastric mucosa in an implanted state of the active implantable medical device. Such an (increased) contact pressure serves - together with the mechanical forces provided by the at least one passive fixation element and optionally the supportive fixation elements - for a secure fixation of the active implantable medical device at the gastric mucosa without introducing any parts of the active implantable medical device into the gastric wall.

In an embodiment, the Nitinol element is provided in form of a Nitinol wire. In its relaxed state, it is shaped as straight or only slightly bent wire. Since the relaxed state cannot be fully achieved by such Nitinol wire within the stomach of the patient, a constant contact pressure is achieved pressing the active implantable medical device against the gastric wall.

In an embodiment, the contact pressure is constant independent on gastric curvature or on a filling state of the stomach.

In an embodiment, a distal region of the elongated electrode body located between the distal end of the elongated electrode body and a central portion of the elongated electrode body has a special shape with respect to a simple elongated shape. To be more precise, the distal region of the elongated electrode body has, in this embodiment, a bifurcated shape, a curved shape, and/or a circular shape.

The bifurcated shape results in at least two distal ends of the elongated electrode body and thus in at least two distal ends of the active implantable medical device itself. To give an example, the bifurcation might result in 2, 3 or 4 bifurcations leading to 2, 3 or 4 distal ends of the elongated electrode body and of the active implantable medical device itself.

The curved shape typically forms a curve having a diameter lying within a range of 2 cm to 20 cm, in particular of 3 cm to 17 cm, in particular of 4 cm to 15 cm, in particular of 5 cm to 12 cm, in particular of 7 cm to 10 cm. By such a curved shape, specific gastric anatomies can be appropriately adapted.

The zigzag shape of the distal end region of the elongated electrode body results in a higher stability of the distal end region and may allow a particularly appropriate electric contact between the elongated electrode body and the gastric mucosa for special gastric anatomies.

The circular shape of the distal end region results in the elongated electrode body contacting itself with its terminal and at a more proximal region of the elongated electrode body. In an embodiment, the circular shape may not result in a full circle, but rather in a shape approximating a full circle. Furthermore, it is generally possible that the circular shape of the distal region of the elongated electrode body comprises a further bar located inside an interior region of a circular shape. The overall appearance of the distal region of the elongated electrode body remains, in this embodiment, a circular shape.

In an embodiment, the memory unit comprises a computer readable program that causes the processor to perform the steps explained in the following when executed on the processor. First, cardiac signals of the patient are detected with the detection unit. In this context, the active implantable medical device has been implanted into the stomach of the patient beforehand. Prior to, concomitantly with or after detecting the cardiac signals, also gastric signals of the patient are detected with the detection unit. Finally, the detected gastric signals are subtracted from the detected cardiac signals so as to obtain pure cardiac signals without any gastric interference signals. Such a procedure results in a higher signal-to-noise ratio of the cardiac signals. This method is a particularly appropriate way to enable detecting highly reliable cardiac signals with an active implantable medical device that can be implanted without any surgical steps (namely, by implanting it into the stomach of the patient, wherein the site of implantation is accessible wire the esophagus).

In an embodiment, the active implantable medical device comprises a data communication unit. Such data communication unit serves for transferring data to and/receiving data from an external evaluation unit or another medical appliance.

In an embodiment, the data communication unit serves for transferring data to the evaluation unit or to another medical appliance in a wireless manner. All standard data transmission protocols or specifications are appropriate for such a wireless data communication. Examples of standard data transmission protocols or specifications are the Medical Device Radiocommunications Service (MICS), the Bluetooth Low Energy (BLE) protocol and the Zigbee specification.

In an embodiment, the data communication unit comprises a communication antenna being operatively coupled with the processor or a control device of the active implantable medical device. The communication antenna can be realized in form of a bipolar loop antenna or a monopole antenna located within the elongated electrode body.

In an embodiment, the data communication unit is used for connecting the active implantable medical device with a subcutaneously implantable cardioverter/defibrillator. Then, it is possible to control or trigger the cardioverter/defibrillator by the active implantable medical device and/or to control or trigger the active implantable medical device by the cardioverter/defibrillator.

In an embodiment, the active implantable medical device comprises an inductive element for inductively receiving energy from a remotely positioned charging device. The received energy is then transmitted to a rechargeable battery of the active implantable medical device which is typically located within a housing of the active implantable medical device. Such an inductive element thus enables a contactless recharge of a rechargeable battery. In an embodiment, the inductive element allows for establishing a radiofrequency coupling in a frequency range of from 1 MHz to 15 MHz, in particular of from 2 MHz to 14 MHz, in particular of from 3 MHz to 13 MHz, in particular of from 4 MHz to 12 MHz, in particular of from 5 MHz to 11 MHz, in particular of from 6 MHz to 10 MHz, in particular of from 7 MHz to 9 MHz. The charging device can be located outside the body of the patient and is typically arranged for charging purposes near the stomach of the patient in which the active implantable medical device is implanted.

In an embodiment, the active implantable medical device comprises a further detection unit. This further detection unit is arranged and designed to detect an impedance, a pH value, a pressure, a force, an acceleration, a salt and/or glycose content, a conductivity and/or a position. The further detection unit can be arranged, e.g., at a position of the active implantable medical device being located near to the pyloric antrum of the stomach of the patient in an implanted state of the active implantable medical device. In an embodiment, the further detection unit serves for detecting an arrival of food in the stomach and to initiate in a time-delayed manner stimulations of the gastric wall. Such stimulations typically trigger a pre-major saturation feeling and may thus cause the patient to stop the uptake of further food. Thus, the patient may stop eating although she or he would have eaten more without the active implantable medical device. A possible application of this functionality of the active implantable medical device is the treatment of obesity.

In an embodiment, the active implantable medical device comprises a stimulation unit configured to stimulate an organ of the patient. As already outlined above, such stimulation unit may be used to stimulate the gastric wall of the patient so as to achieve a pre-major saturation feeling. Furthermore, an insufficient gastric activity may be at least partially compensated or supported by the stimulation unit. This may help in treating gastroparesis. In another application, the stimulation unit can be used to apply cardiac pulses to the heart of the patient.

In an embodiment, the active implantable medical device is used, in operation, for after-shock pacing. For this purpose, an implantable cardioverter/defibrillator may connect itself with the active implantable medical device with the help of the data communication unit of the active implantable medical device. Then, it is possible that the implantable cardioverter/defibrillator informs the active implantable medical device that a cardiac shock has been applied. Subsequently, the active implantable medical device starts an after-shock stimulation of the heart. Alternatively, the active implantable medical device may detect itself the strongly increased electrical fields due to a shock provided by the implantable cardioverter/defibrillator and may thus initiate on its own motion an after-shock pacing of the heart.

In a further embodiment, the active implantable medical device is used for supplying pacing pulses for a regular stimulation of the heart. Alternatively, the active implantable medical device may be used for applying anti-tachycardic pulses having a higher energy to the heart.

The stimulation unit typically makes use of the same electrodes and electrode poles as the detection unit does.

In an aspect, the present invention relates to the further medical use of the active implantable medical device according to the preceding explanations in in-vivo diagnostics of cardiac and/or gastric activity and/or for use in therapy.

In an aspect, the present invention relates to the further medical use of the active implantable medical device according to the preceding explanations in treating gastroparesis or obesity.

In an aspect, the present invention relates to a medical method for performing in-vivo diagnostics of cardiac and/or gastric activity in a patient in need thereof and/or for a cardiac and/or gastric therapy of a patient with the active implantable medical device according to the preceding explanations.

In an aspect, the present invention relates to a medical method for treating gastroparesis or obesity with the active implantable medical device according to the preceding explanations.

In an aspect, the present invention relates to a medical method for implanting an active implantable medical device according to the preceding explanations. For such implanting method, a standard gastroscope can be used. Such a gastroscope typically comprises a light source, an optical channel for a camera or a CCD chip and one or more working channels. The working channels typically have a diameter of 2 to 15 mm. The outer diameter of such a gastroscope typically amounts to 5 to 20 mm.

In an embodiment, a diameter of the active implantable medical device lies within a range of 3 to 6 mm, in particular of 4 to 5 mm so that the medical device can be pushed through the working channel of a standard gastroscope. The gastroscope is then guided through the esophagus into the stomach. There, it releases the active implantable medical device from its working channel. In doing so, the distal end of the active implantable medical device is placed in the gastric fundus. The gastroscope is then moved so as to release the remaining parts of the active implantable medical device, wherein the proximal end of the active implantable medical device is released near the pyloric antrum of the stomach.

In an aspect, the present invention relates to an implantation of the active implantable medical device according to the preceding explanations with the help of the catheter with an inner diameter being sufficiently big to house the medical device, i.e., lying within a range of 4 to 6 mm. The implantation by such a catheter can be monitored by a gastroscope.

Any repositioning of the active implantable medical device can be carried out by forceps being pushed through the working channel of the gastroscope.

In an embodiment, the implantation of the active implantable medical device is monitored by fluoroscopy.

Summarizing, the present invention enables, in an aspect, very easy placement of an active implantable medical device into the stomach of a patient under visual control that can be compared with a gastroscopy. Once implanted, the active implantable medical device ensures a continuous monitoring of the cardiac activity and/or the gastric activity. The replacement of the medical device is possible in a riskless manner. The fixation of the medical device is effected in a purely passive way and is thus particularly gentle for the gastric tissue.

All embodiments of the explained active implantable medical device can be combined in any desired way and can be transferred either individually or in any arbitrary combination to the described uses and/or the described medical methods, and vice versa.

Further details of aspects of the present invention will be explained in the following making reference to exemplary embodiments and accompanying Figures. In the Figures:
- Figure 1: shows a schematic depiction of an active implantable medical device in its intended implantation position;
- Figure 2: shows the implantable medical device of Figure 1 with an implantable cardioverter/defibrillator;
- Figure 3A: schematically shows a portion of a gastroscope being appropriate for implanting the active implantable medical device shown in Figure 1;
- Figure 3B: shows a step of an embodiment of an implantation procedure of an active implantable medical device;
- Figure 4: shows a step of an alternative implantation method of an active implantable medical device;
- Figure 5A: shows a first further embodiment of an active implantable medical device;
- Figure 5B: shows a second further embodiment of an active implantable medical device;
- Figure 5C: shows a third further embodiment of an active implantable medical device;
- Figure 5D: shows details of an embodiment of an implantable medical device;
- Figure 6A: shows an embodiment of an active implantable medical device in its implanted state;
- Figure 6B: shows an enlargement of a portion of the depiction of Figure 6A;
- Figure 7A: shows a first variant of a distal end region of an electrode body of an active implantable medical device;
- Figure 7B: shows a second variant of a distal end region of an electrode body of an active implantable medical device;
- Figure 7C: shows a third variant of a distal end region of an electrode body of an active implantable medical device;
- Figure 7D: shows a fourth variant of a distal end region of an electrode body of an active implantable medical device;
- Figure 7E: shows a fifth variant of a distal end region of an electrode body of an active implantable medical device;
- Figure 7F: shows a sixth variant of a distal end region of an electrode body of an active implantable medical device; and
- Figure 7G: shows a seventh variant of a distal end region of an electrode body of an active implantable medical device.

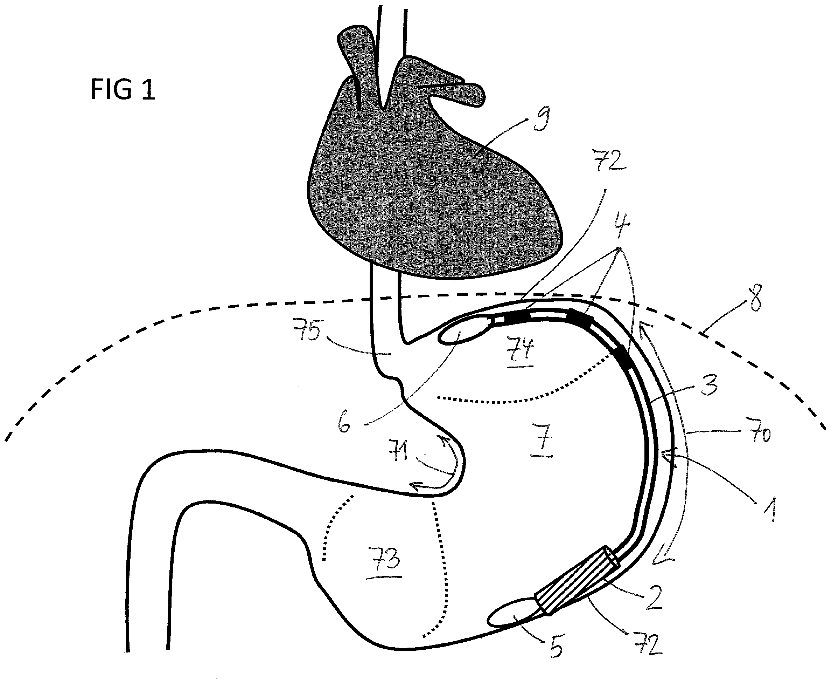

Figure 1 shows a gastric sensor 1 serving as active implantable medical device with a housing 2 and an electrode body 3 as well as three electrode poles 4 arranged on the electrode body 3. The gastric sensor 1 further comprises a first passive fixation element 5 and a second passive fixation element 6. The first passive fixation element 5 is located proximal to the housing 2 at a proximal end of the gastric sensor 1. The second passive fixation element 6 is located at a distal end of the gastric sensor 1 (being at the same time the distal end of the electrode body 3).

The gastric sensor 1 is located within a stomach 7 of a patient along the greater curvature 70 (which, in turn, is located opposite the lesser curvature 71 of the stomach 7). The first passive fixation element 5 is located at a caudal portion of the stomach wall 72 near the pyloric antrum 73. The distal fixation element is located at a cranial portion of the stomach wall 72 in the fundus 74 near the cardia 75. Due to this arrangement of the gastric sensor 1, the electrode poles 4 are also located at a cranial portion of the stomach wall 72. Thus, they are closely positioned beneath the diaphragm 8 and are also in close proximity to the heart 9 of the patient. Therefore, it is generally possible to detect cardiac signals with the electrode poles 4 and thus with the gastric sensor 1.

The gastric sensor 1 presses with an intrinsic pre-tensioning against the gastric wall 72. The first passive fixation element 5 and the second passive fixation element 6 serve for a purely passive fixation of the gastric sensor 1 at or within the gastric mucosa of the gastric wall 72.

Even though the embodiment depicted in Figure 1 is intended to be used for detecting cardiac signals with the electrode poles 4 of the gastric sensor 1, the gastric sensor 1 can be likewise used for detecting gastric signals (e.g., being representative for gastric activity).

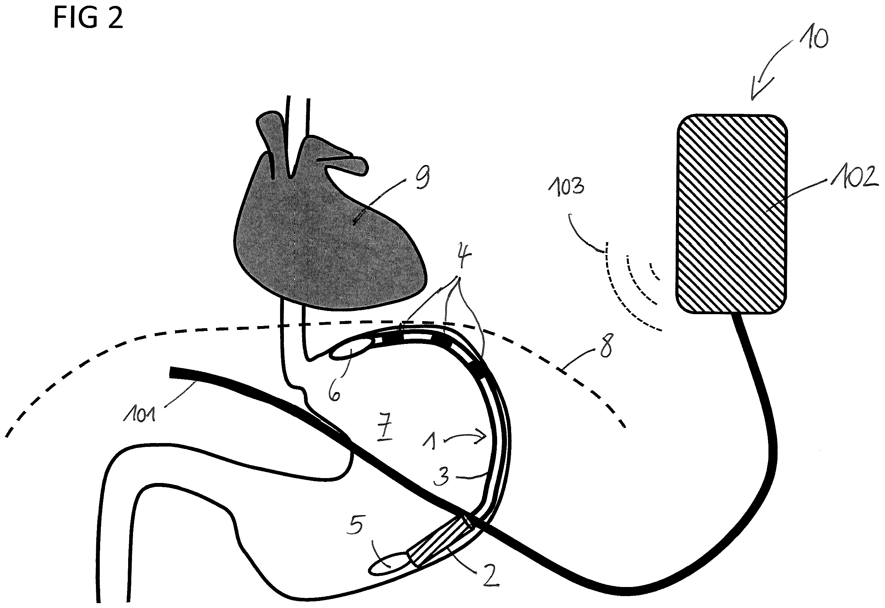

Figure 2 shows a similar arrangement like Figure 1. In this and in all following Figures, similar elements will be denoted with the same numeral references.

In addition to the arrangement shown in Figure 1, the arrangement of Figure 2 additionally comprises an implantable cardioverter/defibrillator 10 that has been subcutaneously implanted to the body of the patient. The cardioverter/defibrillator 10 comprises an electrode 101 and a housing 102 serving as counter electrode. In operation, the cardioverter/defibrillator 10 can emit shock waves 103 for applying a defibrillation therapy to the heart 9.

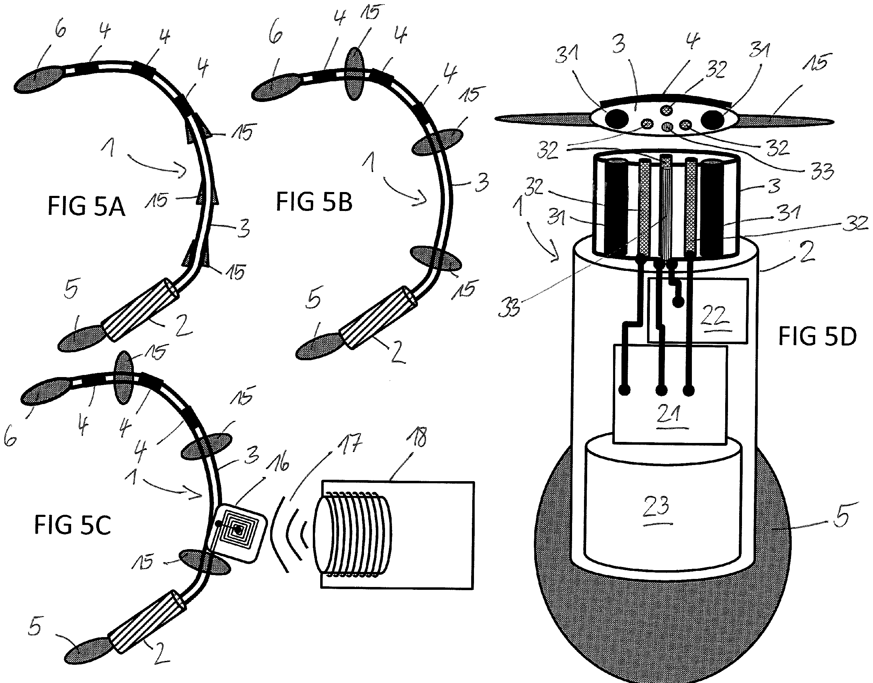

Afterwards, the gastric sensor 1 can be used for applying after-shock pacing to the heart 9. For this purpose, the gastric sensor also comprises a stimulation unit, parts of which are contained in the housing 2 of the gastric sensor 1. The application of such after-shock pacing can be initiated by a detection of the strong electric field due to the shockwave 103 or by a direct communication between the cardioverter/defibrillator 10 and the gastric sensor 1 by means of a data communication unit comprising an antenna (cf. Figure 5 D for more details).

Thus, the gastric sensor 1 can interact with other implants within the body of the patient so as to ameliorate a therapy provided to the patient.

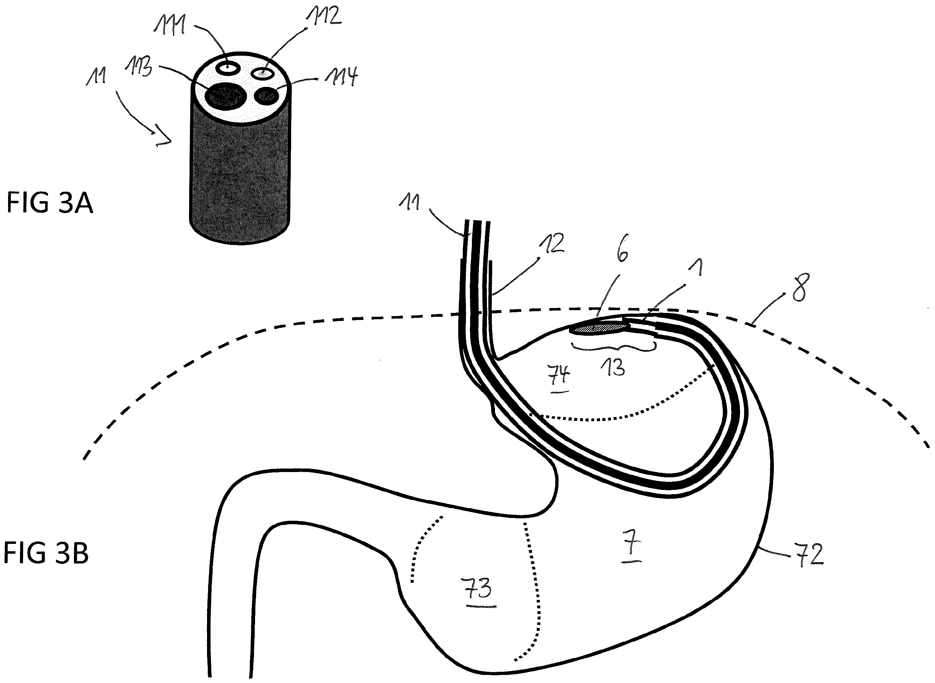

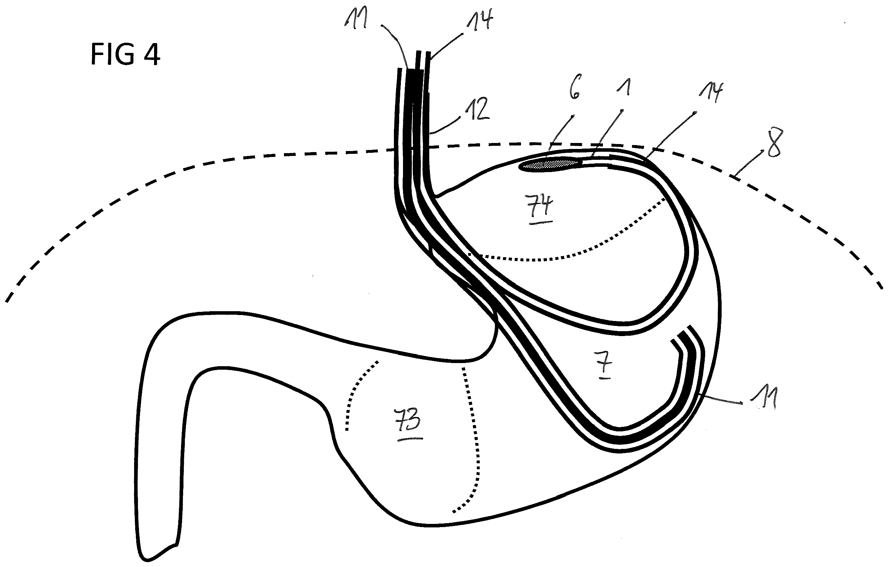

The gastric sensor 1 as illustrated in Figures 1 and 2 can be particularly easy implanted into the stomach 7 of the patient. This will be explained in more detail making reference to Figures 3A, 3B and 4.

Figure 3B shows a schematic depiction of a portion of a standard gastroscope 11. The gastroscope 11 comprises a light source 111, an optical channel 112 for a camera or a CCD chip, and two working channels 113, 114. The working channels 113, 114 typically have an inner diameter lying in a range between 2 and 15 mm. The gastric sensor 1 (cf. Figures 1 and 2) is typically sized such to have an outer diameter lying in a range of from 3 to 5 mm. Then, it can be easily inserted into one of the working channels 113, 114 of the standard gastroscope 11.

The implantation of the gastric sensor 1 is particularly simple, as can be seen in Figure 3B. The gastroscope 11 is guided through the esophagus 12 into the stomach 7 of the patient. Here, the distal end 13 with the second passive fixation element 6 is released from the gastroscope 11 in the fundus 74 of the stomach 7 of the patient. The gastroscope 11 is then continuously retracted so as to also release the other portions of the gastric sensor 1. Finally, the proximal portion of the gastric sensor 1 with the first passive fixation element 5 (cf. Figures 1 and 2 for more details) will be released at the stomach wall 72 near the pyloric antrum 73.

Another possibility of implanting the gastric sensor 1 into the stomach 7 of the patient is depicted in Figure 4. Here, a catheter 14 is used in addition to the gastroscope 11. Both the gastroscope 11 and the catheter 14 are guided through the esophagus 12 into the stomach 7 of the patient. Here, the gastric sensor 1 is released from the catheter 14 in the fundus 74 of the stomach 7, as in case of an implantation with a gastroscope explained with respect to Figure 3B. The gastroscope 11 is used in the embodiment of Figure 4 to monitor the implantation of the gastric sensor 1 with the help of the catheter 14.

Figures 5A to 5C show further embodiments of the gastric sensor 1. In these embodiments, the gastric sensor 1 comprises additional or supportive passive fixation elements 15 disposed along the electrode body 3 of the gastric sensor 1. In the embodiment of Figure 5A, the supportive fixation elements 15 have a wing-like shape. In the embodiments of Figures 5B and 5C, the supportive passive fixation elements 15 have a disc-like shape. The supportive passive fixation elements 15 serve for an additional passive fixation of the gastric sensor 1 within the gastric mucosa of the patient. Then, the forces being applied between the gastric sensor 1 and the gastric mucosa of the patient are more evenly distributed than in case of using only the proximal passive fixation element 5 and/or the distal passive fixation element 6.

The embodiment of the gastric sensor 1 shown in Figure 5C additionally comprises a spirally shaped conductive structure 16 serving as inductive element. The conductive structure 16 is made from a plastic foil into which a conductive element is embedded. The conductive structure 16 serves for receiving high frequency energy 17 having a frequency of 13 MHz emitted from a coil 18 located outside the patient. In doing so, it is possible to transfer energy from outside the patient into the gastric sensor 1. This energy is used to recharge a rechargeable battery located within the housing 2 and used for the energy consuming tasks of the gastric sensor 1.

Figure 5D shows a partially cut lateral view as well as a cross-sectional view of an embodiment of the gastric sensor 1. The cross-sectional view on top of Figure 5D shows an oval shaped electrode body 3 in the area of an electrode pole 4. The electrode pole 4 is only arranged at that side of the electrode body 3 that faces the gastric mucosa upon implantation of the gastric sensor 1. Furthermore, a supportive passive fixation element 15 can be seen, the function of which was explained with respect to Figures 5A to 5C.

The oval shape of the electrode body 3 serves for an easier orientation of the electrode body 3 with respect to the gastric mucosa. In its interior, the electrode body 3 comprises two Nitinol wires 31 that serve for stiffening the electrode body 3 and for applying a pre-tensioning to the electrode body 3. This increases the contact pressure of the electrode body 3 with respect to the gastric mucosa at which the gastric sensor 1 is to be implanted. The interior of the electrode body 3 further comprises three wire-like conductors 32 that enable an electric contact between the electrode poles 4 and a memory unit. Furthermore, the electrode body 3 comprises an antenna 33 for sending and/or receiving data.

The Nitinol wires 31, the wire-like conductors 32 and the antenna 33 can also be seen in the lateral view of Figure 5D. Here, it becomes apparent that the 3 wire-like conductors or conductive wires 32 are connected to a memory unit 21 located inside the housing 2 of the gastric sensor 1. Due to this connection, electrical signals sensed by the electrode poles 4 can be guided through the conductive wires 32 towards the memory unit 21 and stored within the memory unit 21 until further usage. The antenna 33 is connected to a communication unit 22 also located within the housing 2. Furthermore, a control unit and a processor (not shown) are located within the housing 2. All active elements are supplied with energy by a rechargeable battery 23 also located within the housing 2. As explained with respect to the embodiment shown in Figure 5C, it is generally possible to recharge the battery 23 from an outside of the patient in an inductive manner.

Proximal from the housing 2, the first passive fixation element 5 is arranged. It also serves for protection of the gastric mucosa against any injuries that might occur due to the presence of sharp edges on the housing 2.

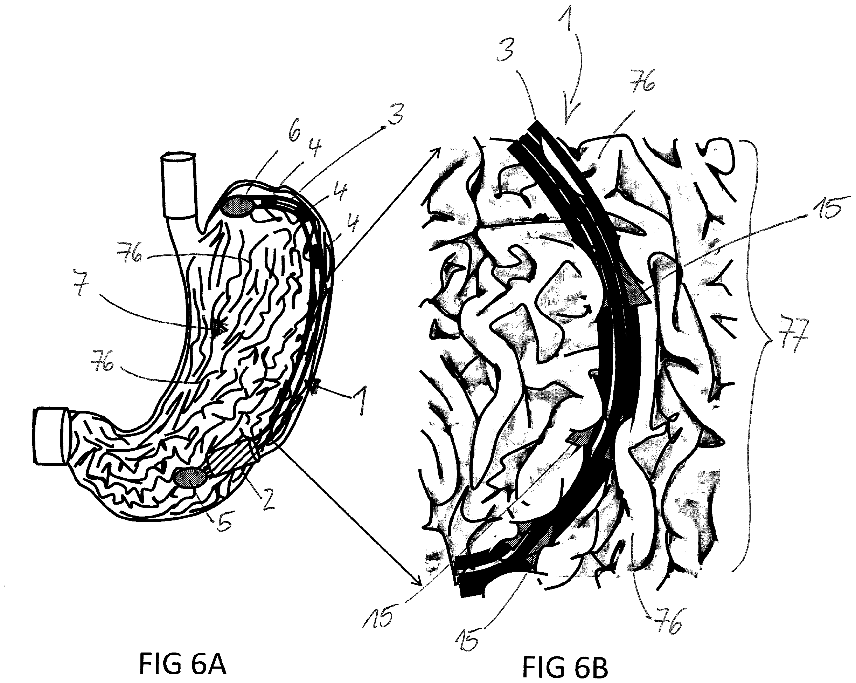

Figure 6A shows a more detailed view on the concrete implantation position of the gastric sensor 1 within the stomach 7. Here, gastric rugae 76 of the gastric mucosa 77 (cf. Figure 6B) can be seen.

Figure 6B shows an enlarged view of a portion of Figure 6A. Here, it is visible that the supportive passive fixation elements 15 of the gastric sensor 1 tightly (but passively) interact with the gastric rugae 76 of the gastric mucosa 77. Due to this passive interaction, an undesired dislocation of the gastric sensor is efficiently prevented. At the same time, irritations of the gastric mucosa 77 due to the implantation of the gastric sensor 1 are reduced to a minimum since no penetration of the gastric mucosa 77 nor of the gastric wall is necessary for safely fixating the gastric implant 1 within the stomach.

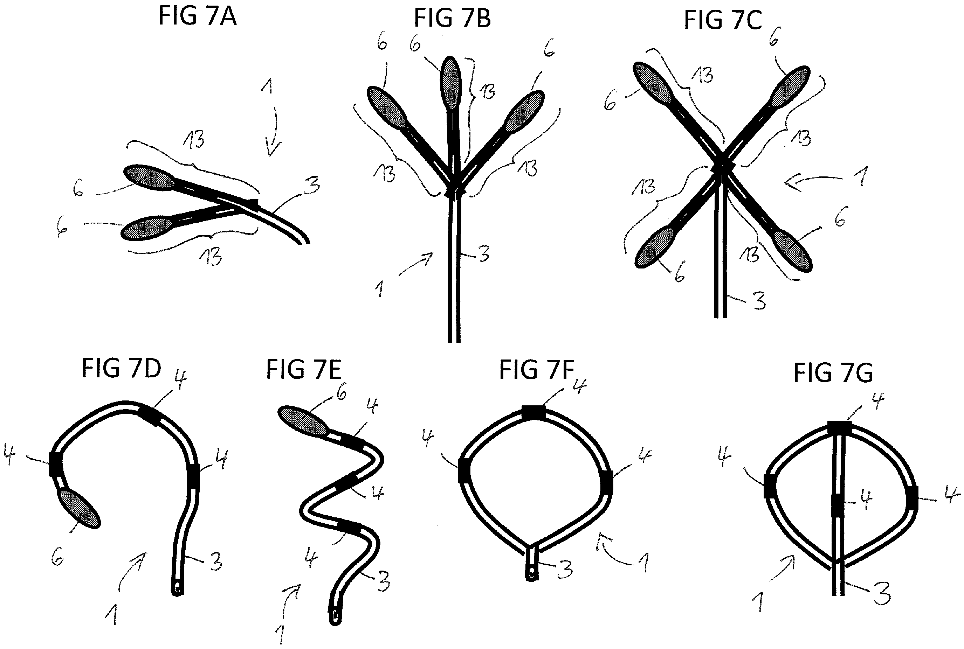

Figures 7A to 7G show embodiments of the distal end region of the gastric sensor 1 referred to in the preceding Figures.

According to the embodiment shown in Figure 7A, the electrode body 3 of the gastric sensor 1 is bifurcated so as to have two distal ends 13 with two passive fixation elements 6.

According to the embodiment shown in Figure 7B, the electrode body 3 of the gastric sensor 1 is bifurcated so as to have three distal ends 13 with three passive fixation elements 6.

According to the embodiment shown in Figure 7C, the electrode body 3 of the gastric sensor 1 is bifurcated so as to have four distal ends 13 with four passive fixation elements 6.

In the embodiment shown in Figure 7D, the distal end section of the gastric sensor has a curved shape, wherein the formed curve has a diameter of approximately 10 cm. The electrode poles 4 are disposed within this curved shape.

In the embodiment shown in Figure 7E, the electrode body 3 of the gastric sensor 1 has a zigzag shape, wherein the electrode poles 4 are arranged in the zigzag shaped portion of the electrode body 3.

In the embodiment shown in Figures 7F and 7G, the distal end portion of the gastric sensor 1 has a circular shape, wherein the distal end of the electrode body 3 contacts a more proximately located portion of the electrode body 3. The electrode poles 4 are disposed in the circle formed by this circular end shape of the gastric sensor 1.

In Figure 7 G, an additional central bar is formed within the circle of the circular shape.

In the embodiments shown in Figures 7F and 7G employing a circular shape of the distal end region of the gastric sensor 1, no second passive fixation element 6 is provided. This is due to the fact that the circular shape of the distal end region of the gastric sensor 1 already serves itself for particularly tissue-gentle properties of the gastric set sensor 1. Furthermore, due to the circular shape of the distal region of the gastric sensor 1, the distal region of the gastric sensor 1 is no longer well suited for passive fixation within the gastric mucosa. Thus, in these embodiments, the passive fixation is only achieved with the first passive fixation element 5 located at the proximal end of the gastric sensor 1 (cf. Figures 1 and 2 for more details) and optionally by supportive passive fixation elements 15 (see Figures 5A and 5B for more details) as well as optionally by a Nitinol wire 31 (cf. Figure 5D for more details).

## Claims

1. Active implantable medical device for sensing an electric signal of an organ of a human or animal patient, comprising a processor, a memory unit (21), and a detection unit configured to detect an electric signal of an organ of a human or animal patient, wherein the detection unit comprises an elongated electrode body (3) extending between a proximal end and a distal end (13) of the active implantable medical device (1) and having at least one electrode pole (4), wherein the active implantable medical device (1) is configured to be implanted within a stomach (7) of the patient, the stomach (7) having a cardia (75), a fundus (74), a greater curvature (70), a lesser curvature (71), a pyloric antrum (73), and a stomach wall (72) with gastric mucosa (77) facing an interior of the stomach, with the elongated electrode body (3) being placed along the greater curvature (70),
**characterized**
**in that** the active implantable medical device (1) comprises a passive fixation element (5, 6) at at least one of the proximal end and the distal end (13) of the active implantable medical device (1), wherein the passive fixation element (5, 6) serves for a purely passive fixation of the active implantable medical device (1) at the gastric mucosa (77).

2. Active implantable medical device according to claim 1, **characterized in that** the passive fixation element (5) is positioned at the proximal end of the active implantable medical device (1).

3. Active implantable medical device according to claim 1, **characterized in that** the active implantable medical device (1) comprises a first passive fixation element (5) at the proximal end of the active implantable medical device (1) and a second passive fixation element (6) at the distal end (13) of the active implantable medical device (1).

4. Active implantable medical device according to any of the preceding claims, **characterized in that** the active implantable medical device (1) comprises a housing (2) accommodating the processor and the memory unit (21) and being operatively coupled to the elongated electrode body (3), wherein the housing (2) is positioned between a proximal end of the elongated electrode body (3) and the proximal end of the active implantable medical device (1).

5. Active implantable medical device according to any of the preceding claims, **characterized in that** the elongated electrode body (3) comprises at least two electrode poles (4) being spaced apart from each other by at least 10 mm.

6. Active implantable medical device according to any of the preceding claims, **characterized in that** the active implantable medical device (1) comprises at least one supportive passive fixation element (15) disposed on the elongated electrode body (3), wherein the supportive passive fixation element (15) serves for an additional purely passive fixation of the active implantable medical device (1) at the gastric mucosa (77).

7. Active implantable medical device according to any of the preceding claims, **characterized in that** the elongated electrode body (3) comprises at least one Nitinol element (31) serving, in an implanted state of the active implantable medical device (1), for providing a contact pressure of the elongated electrode body (3) against the gastric mucosa (77).

8. Active implantable medical device according to any of the preceding claims, **characterized in that** a distal region of the elongated electrode body (3) located between a distal end (13) of the elongated electrode body (3) and a central portion of the elongated electrode body (3) has at least one of a bifurcated shape resulting in at least two distal ends (13) of the elongated electrode body (3) and of the active implantable medical device (1), a curved shape forming a curve with a diameter lying within a range of 2 cm to 20 cm, a zig-zag shape, and a circular shape.

9. Active implantable medical device according to any of the preceding claims, **characterized in that** the memory unit (21) comprises a computer-readable program that causes the processor to perform the following steps when executed on the processor: a) detecting, with the detection unit, cardiac signals of a patient into the stomach (7) of whom the active implantable medical device (1) has been implanted; b) detecting, with the detection unit, gastric signals of the patient; c) subtracting the detected gastric signals from the detected cardiac signals.

10. Active implantable medical device according to any of the preceding claims, **characterized in that** the active implantable medical device (1) comprises a data communication unit (22) for transferring data to and/or receiving data from an external evaluation unit.

11. Active implantable medical device according to any of the preceding claims, **characterized in that** the active implantable medical device (1) comprises an inductive element (16) for inductively receiving energy from a remotely positioned charging device and for transmitting the received energy to a rechargeable battery (23) of the active implantable medical device (1).

12. Active implantable medical device according to any of the preceding claims, **characterized in that** the active implantable medical device (1) comprises a further detection unit configured to detect at least one of an impedance, a pH value, a pressure, a force, an acceleration, a salt and/or glycose content, a conductivity and a position.

13. Active implantable medical device according to any of the preceding claims, **characterized in that** the active implantable medical device (1) comprises a stimulation unit configured to stimulate an organ of the patient.

14. Active implantable medical device according to any of the preceding claims for use in in vivo diagnostics of cardiac and/or gastric activity or for use in therapy.

15. Active implantable medical device according to any of claims 1 to 13 for use in treating gastroparesis or obesity.
